# EUROPÄISCHE PATENTSCHRIFT

(11) **EP 1 070 079 B1**
(45) Veröffentlichungstag und Bekanntmachung des Hinweises auf die Patenterteilung: **18.02.2004**
(21) Anmeldenummer: 99919205.7
(22) Anmeldetag: 07.04.1999
(51) Int. Cl.: C07H 21/00, C07H 19/04

(54) **VERFAHREN ZUR HERSTELLUNG VON PENTOPYRANOSYL-NUCLEOSIDEN**
METHOD FOR THE PRODUCTION OF PENTOPYRANOSYL NUCLEOSIDES
PROCEDE DE PREPARATION DE PENTOPYRANOSYLE-NUCLEOTIDES

(30) Priorität: 08.04.1998 DE 19815901
(43) Veröffentlichungstag der Anmeldung: 24.01.2001
(73) Patentinhaber: Nanogen Recognomics GmbH, 65926 Frankfurt am Main (DE)
(72) Erfinder: ESCHENMOSER, Albert, CH-8700 Küsnacht (CH); PITSCH, Stefan, CH-8049 Zürich (CH); WENDEBORN, Sebastian, CH-4102 Binningen (CH)
(74) Vertreter: Ackermann, Joachim, Dr.
(86) Internationale Anmeldenummer: PCT/EP1999/002356
(87) Internationale Veröffentlichungsnummer: WO 1999/052923

(56) Entgegenhaltungen:
- WO-A-99/15540
- ESCHENMOSER ET AL: "147. Why pentose- and not hexose-nucleic acids?" HELVETICA CHIMICA ACTA, Bd. 76, 1. Januar 1993 (1993-01-01), Seiten 2161-2183, XP002094190 ISSN: 0018-019X in der Anmeldung erwähnt
- M BHRINGER ET AL: "110. Warum Pentose- und nicht Hexose-Nucleins uren ? Oligonucleotide aus 2',3'-Dideoxy-B-D-glucopyranosyl-Bausteine n ('Homo-DNS?): Herstellung" HELVETICA CHIMICA ACTA, Bd. 75, Nr. 5, 1. Januar 1992 (1992-01-01), Seiten 1416-1477, XP002096856 ISSN: 0018-019X in der Anmeldung erwähnt
- H.VORBRÜGGEN ET ALL.: "Nucleoside Synthesis with Trimethylsilyl Triflate and Perchlorate as Catalysts." CHEMISHE BERICHTE, Bd. 114, 1981, Seiten 1234-1255, XP002121064 in der Anmeldung erwähnt

## Beschreibung

Die vorliegende Erfindung betrifft ein Verfahren zur Herstellung eines 3', 4'cyclischen Acetals eines Pentopyranosyl-Nucleosids, bei dem ein Pentopyranosyl-Nucleosid mit einem Aldehyd, Keton, Acetal oder Ketal unter Unterdruck umgesetzt wird.

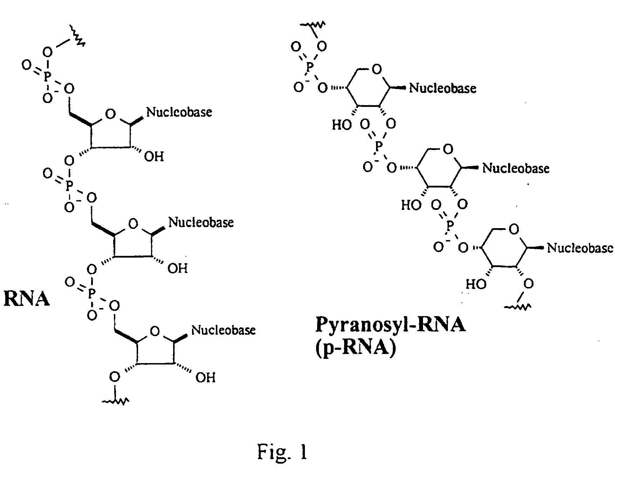

Pyranosyl-Nucleinsäuren (p-NA's) sind im allgemeinen zur natürlichen RNA isomere Strukturtypen, bei denen die Pentose-Einheiten in der Pyranoseform vorliegen und durch Phosphodiestergruppen zwischen den Positionen C-2' und C-4' repetitiv verknüpft sind (Fig. 1). Unter "Nucleobase" werden dabei die kanonischen Nucleobasen A, T, U, C, G, aber auch die Paare Isoguanin/Isocytosin und 2,6-Diaminopurin/Xanthin und im Sinne der vorliegenden Erfindung auch andere Purine und Pyrimidine verstanden. p-NA's, und zwar die von der Ribose abgeleitete p-RNA's, wurden zum erstenmal von Eschenmoser et al. beschrieben (s. S. Pitsch et al., *Helv. Chim. Acta* 76, 2161 (1993); S. Pitsch et al., *Helv. Chim Acta* 78, 1621 (1995); Krishnamurthy, R. et al., *Angew. Chem.* 108, 1619-1623 (1996)). Sie bilden ausschließlich sogenannte Watson-Crick-gepaarte, d.h. Purin-Pyrimidin- und Purin-Purin-gepaarte, antiparallele, reversibel "schmelzende", quasi-lineare und stabile Duplices. Homochirale p-RNA-Stränge entgegengesetzten Chiralitätssinns paaren ebenfalls kontrollierbar und sind in der gebildeten Duplex streng nicht-helical. Diese für den Aufbau supramolekularer Einheiten wertvolle Spezifität hängt mit der relativ geringen Flexibilität des Ribopyranosephosphat-Rückgrats sowie mit der starken Neigung der Basenebene zur Strangachse und der hieraus folgenden Tendenz zu intercatenarer Basenstapelung im resultierenden Duplex zusammen und läßt sich letzlich auf die Teilnahme eines 2',4'-cis-disubstituierten Ribopyranoserings am Aufbau des Rückgrates zurückführen. Diese wesentlich besseren Paarungseigenschaften machen p-NA's gegenüber DNA und RNA für die An wendung des Aufbaus supramolekularer Einheiten zu bevorzugten Paarungssystemen. Sie bilden ein zu natürlichen Nucleinsäuren orthogonales Paarungsystem, d. h. sie paaren nicht mit in der natürlich Form vorkommenden DNA's und RNA's, was im besonderen im diagnostischen Bereich von Bedeutung ist.

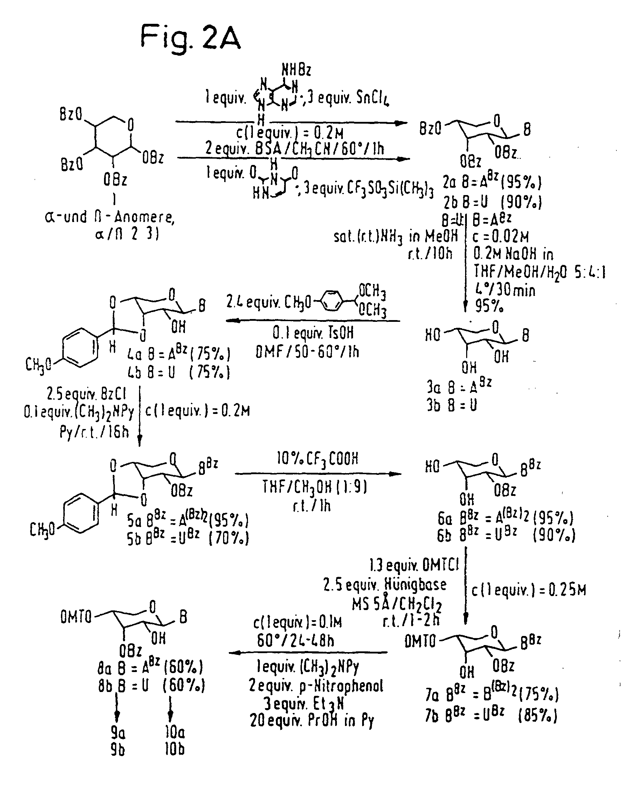

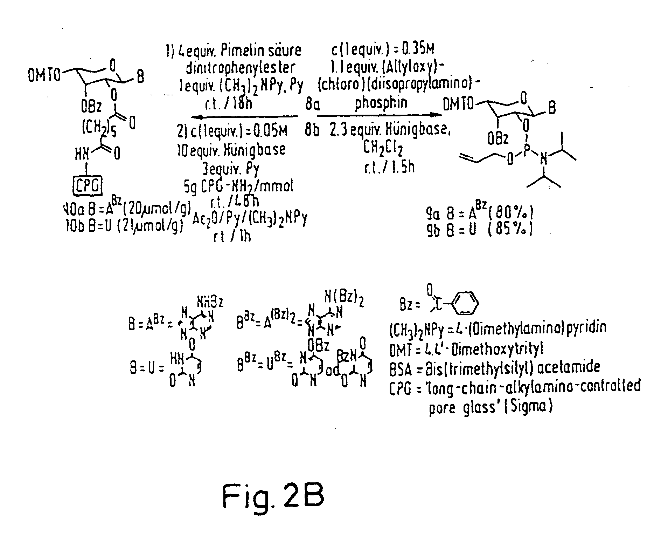

Eschenmoser et al. hat zum ersten Mal eine p-RNA, wie in Fig. 2 dargestellt und nachstehend erläutert, hergestellt (s. auch S. Pitsch et al. (1993), supra)

Hierbei wurde eine geeignete geschützte Nucleobase mit dem Anomerengemisch der Tetrabenzoyl-Ribopyranose durch Einwirken von Bis(trimethylsilyl)acetamid und einer Lewis-Säure wie z. B. Trimethylsilyl-trifluormethansulfonat zur Reaktion gebracht (analog Vorbrüggen, H. et al., *Chem. Ber*. 114, 1234 (1981)). Unter Baseneinwirkung (NaOH in THF/Methanol/Wasser im Falle der Purine; gesättigter Ammoniak in MeOH im Falle der Pyrimidine) wurden die Acylschutzgruppen vom Zucker abgespalten, und das Produkt unter saurer Katalyse mit p-Anisaldehyddimethylacetal in 3',4'-Position geschützt. Das Diastereomerengemisch wurde in 2'-Stellung acyliert, das 3',4'-methoxybenzylidengeschützte 2'-Benzoat durch saure Behandlung, z.B. mit Trifluoressigsäure in Methanol deacetalisiert, und mit Dimethoxytritylchlorid umgesetzt. Die 2'--3'-Wanderung des Benzoats wurde durch Behandlung mit p-Nitrophenol/4-(Dimethylamino)pyridin/Triethylamin/Pyridin/n-Propanol eingeleitet. Fast alle Reaktionen wurden durch Säulenchromatographie aufgearbeitet. Der so synthetisierte Schlüsselbaustein, das 4'-DMT-3'-benzoyl-1'-Nucleobasen-Derivat der Ribopyranose, wurde dann zum Teil phosphityliert bzw. über einen Linker an eine feste Phase gebunden.

Bei der anschließenden automatisierten Oligonucleotidsynthese wurde die trägergebundene Komponente in 4'-Position wiederholt sauer entschützt, ein Phosphoramidit unter Einwirkung eines Kupplungsreagenz, z. B. ein Tetrazolderivat, angekuppelt, noch freie 4'-Sauerstoffatome acetyliert und das Phosphoratom oxidiert, um so das oligomere Produkt zu erhalten. Anschließend wurden die restlichen Schutzgruppen abgespalten, das Produkt über HPLC gereinigt und entsalzt.

Das von Eschenmoser et al. beschriebene Verfahren kann jedoch nicht mit den angegebenen Ausbeuten reproduziert werden und ist somit für eine Anwendung im industriellen Maßstab kaum geeignet.

Aufgabe der vorliegenden Erfindung war daher, ein Verfahren bereitzustellen, das eine Herstellung von Pentopyranosyl-Nucleosiden im industriellen Maßstab ermöglicht.

Es wurde nun überraschenderweise gefunden, daß die Herstellung des 3', 4'cyclischen Acetals eines Pentopyranosyl-Nucleosids, das ein Zwischenprodukt bei der Eschenmoser-Synthese ist, erst dann in nennenswerten Ausbeuten gelingt, wenn das Pentopyranosyl-Nucleosid unter Unterdruck mit einem Aldehyd oder Keton bzw. mit einem Acetal oder Ketal umgesetzt wird.

Ein Gegenstand der vorliegenden Erfindung ist daher ein Verfahren zur Herstellung eines 3', 4'-cyclischen Acetals eines Pentopyranosyl-Nucleosids, bei dem ein Pentopyranosyl-Nucleosid mit einem Aldehyd, Keton, Acetal oder Ketal unter Unterdruck umgesetzt wird.

Unter dem Begriff Unterdruck versteht man gemäß der vorliegenden Erfindung ein Druck von weniger als 500 mbar, vorzugsweise von weniger als 100 mbar, insbesondere von weniger als 50 mbar, vor allem von 30 mbar.

Der Aldehyd ist beispielsweise Formaldehyd, Acetaldehyd, Benzaldehyd oder 4-Methoxybenzaldehyd, das Acetal Formaldehyd-dimethylacetal, Acetaldehyd-dimethylacetal, Benzaldehyd-dimethylacetal oder 4-Methoxybenzaldehyddimethylacetal, das Keton Aceton, Cyclopentanon oder Cyclcohexanon und das Ketal Acetondimethylketal, Cyclopentanon-dimethylketal, Cyclohexanon-dimethylketal oder in Form von 2-Methoxypropen.

In einer besonderen Ausführungsform wird das Pentopyranosyl-Nucleosid vor der Umsetzung beispielsweise über SiO₂ , vorzugsweise über SiO₂ in Form von Kieselgel, gereinigt. Hierzu eignet sich beispielsweise eine Reinigung über eine Kieselgel-Chromatographiesäule. Für die Elutions des Pentopyranosyl-Nucleosids eignet sich z. B. ein Gradient von ca. 1-20% oder ca. 5-15% Methanol in Dichlormethan. Besonders vorteilhaft ist es, wenn das Pentopyranosyl-Nucleosid vor der Reinigung beispielsweise mit einer 1%igen Salzsäurelösung oder mit festem Ammoniumchlorid neutralisiert wird und gegebenenfalls die Lösemittel abgezogen werden..

Als Pentopyranosyl-Nucleosid eignet sich im allgemeinen ein Ribo-, Arabino-, Lyxo- oder Xylo-pyranosyl-Nucleosid. Beispiele von geeigneten Pentopyranosyl-Nucleosiden sind ein Pentopyranosyl-purin, -2,6-diaminopurin, -6-purinthiol, -pyridin, -pyrimidin, -adenosin, -guanosin, -isoguanosin, -6-thioguanosin, -xanthin, -hypoxanthin, -thymidin, -cytosin, -isocytosin , -indol, -tryptamin, -N-phthaloyltryptamin, -uracil, -coffein, -theobromin, -theophyllin, -benzotriazol oder -acridin.

Formelmäßig können die Pentopyranosyl-Nucleoside durch die Formel (I) 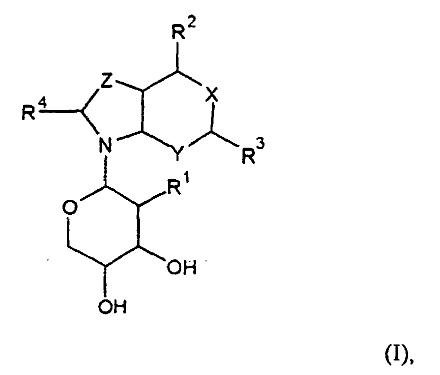 worin
R¹ gleich H, OH oder Hal mit Hal gleich Br oder Cl,
R², R³ und R⁴ unabhängig voneinander, gleich oder verschieden, jeweils H, Hal mit Hal gleich Br oder Cl, NR⁵R⁶, OR⁷, SR⁸, =O, CₙH₂ₙ₊₁ mit n eine ganze Zahl von 1-12, vorzugsweise 1-8, insbesondere 1-4, oder (CₙH₂ₙ)NR¹⁰R¹¹ mit R¹⁰R¹¹ gleich H, CₙH₂ₙ₊₁ oder R¹⁰R¹¹ verbunden über einen Rest der Formel 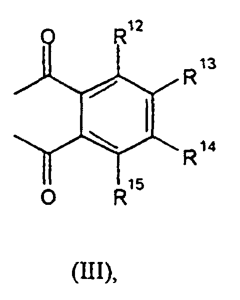 worin R¹², R¹³, R¹⁴ und R¹⁵ unabhängig voneinander, gleich oder verschieden, jeweils H, OR⁷, wobei R⁷ die genannte Bedeutung hat, oder CₙH₂ₙ₊₁, oder CₙH₂ₙ₋₁,
wobei n die oben genannte Bedeutung hat, bedeuten und
R⁵, R⁶, R⁷ und R⁸ unabhängig voneinander, gleich oder verschieden, jeweils H, CₙH₂ₙ₊₁, oder CₙH₂ₙ₋₁, wobei n die oben genannte Bedeutung hat, -C(O)R⁹ mit R⁹ gleich ein linearer oder verzweigter, gegebenenfalls substituierter Alkyl-, Aryl-, vorzugsweise Phenyl-Rest,
X, Y und Z unabhängig voneinander, gleich oder verschieden, jeweils =N-, =C(R¹⁶)- oder -N(R¹⁷)- mit R¹⁶ und R¹⁷ unabhängig voneinander, gleich oder verschieden, jeweils H oder CₙH₂ₙ₊₁ oder (CₙH₂ₙ)NR¹⁰R¹¹ mit den oben genannten Bedeutungen, bedeutet,
oder durch die Formel (II) 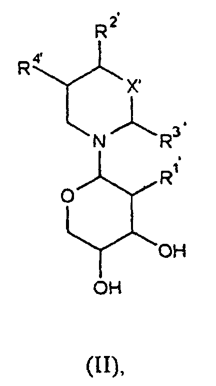 worin R^{1'} gleich H, OH oder Hal mit Hal gleich Br oder Cl,
R^{2'}, R^{3'} und R^{4'} unabhängig voneinander, gleich oder verschieden, jeweils H, Hal mit Hal gleich Br oder Cl, =O, CₙH₂ₙ₊₁ oder OCₙH₂ₙ₋₁, oder (CₙH₂ₙ)NR^{10'}R^{11'},
wobei R^{10'}, R^{11'}, unabhängig voneinander die oben genannte Bedeutung von R¹⁰ bzw. R¹¹ hat, und
X' jeweils =N-, =C(R^{16'})- oder -N(R^{17'})- bedeutet, wobei R^{16'} und R^{17'} unabhängig voneinander die oben genannte Bedeutung von R¹⁶ bzw. R¹⁷ haben, dargestellt werden.

Das erfindungsgemäße Verfahren erfolgt im allgemeinen bei einer Temperatur von ca. 40-70 °C, vorzugsweise von ca. 50-60 °C, insbesondere von ca. 50-55 °C. Ferner wird die Umsetzung im allgemeinen unter saurer Katalyse beispielsweise in Gegenwart von p-Toluolsulfonsäure, Methansulfonsäure, Tetrafluoroborsäure, Schwefelsäure, sauren Ionenaustauscher, wie z. B. saure Amberlite® (Rohm & Haas) und/oder Lewissäuren, wie z. B. Zinkchlorid, Trimethylsilyltriflat oder Pyridiniumparatoluolsulfonat, durchgeführt. Die Reaktionszeiten betragen üblicherweise ca. 1-1,5 Stunden, vorzugsweise ca. 1,5 Stunden.

In einer weiteren Ausführungsform des erfindungsgemäßen Verfahrens kann in einem weiteren Schritt das gemäß dem obigen Verfahren erhaltene 3', 4'cyclische Acetal eines Pentopyranosyl-Nucleosids an der 2'-Position geschützt werden. Vorzugsweise wird die 2'-Position durch eine basenlabile oder metallkatalysiert abspaltbare Schutzgruppe, insbesondere durch eine Acylgruppe, vor allem durch eine Acetyl-, Benzoyl-, Nitrobenzoyl- und/oder Methoxybenzoylgruppe, nach dem Fachmann bekannten Verfahren beispielsweise mit Benzoylchlorid in einer Dimethylaminopyridin/Pyridin-Lösung bei Raumtemperatur geschützt.

In einer weiteren Ausführungsform des erfindungsgemäßen Verfahrens kann das an der 2'-Position geschützte 3', 4'-cyclische Acetal eines Pentopyranosyl-Nucleosids deketalisiert werden. Im allgemeinen erfolgt die Deketalisierung in Gegenwart einer Säure, vorzugsweise in Gegenwart einer starken Säure, wie z. B. Trifluoressigsäure. Die Aufarbeitung des erhaltenen Reaktionsproduktes erfolgt vorzugsweise trocken-basisch, beispielsweise in Anwesenheit von festem Hydrogencarbonat, Carbonat und/oder basischem Ionenaustauscher, wie z. B. basische Amberlite® (Rohm & Haas). Anschließend kann das aufgearbeitete Reaktionsprodukt beispielsweise über SiO₂, insbesondere über SiO₂ in Form von Kieselgel, gereinigt werden.

In einer weiteren Ausführungsform des erfindungsgemäßen Verfahrens kann in einem weiteren Schritt auch die 4'-Position geschützt werden. Als Schutzgruppe eignet sich im allgemeinen eine säure- oder basenlabile Schutzgruppe, vorzugsweise eine Trityl-Gruppe, insbesondere eine DMT-Gruppe, und/oder eine β-eliminierbare Gruppe, insbesondere eine Fmoc-Gruppe. Die Einführung einer Schutzgruppe erfolgt nach allgemein bekannten Verfahren beispielsweise durch Dimethoxytritylchlorid in Gegenwart von z. B. N-Ethyldiisopropylamin (Hünig-Base).

In einer weiteren Ausführungsform des erfindungsgemäßen Verfahrens kann in einem weiteren Schritt eine Umlagerung der Schutzgruppe von der 2'-Position zur 3'-Position erfolgen. Im allgemeinen wird die Umlagerung in Gegenwart einer Base, insbesondere in Gegenwart von N-Ethyldiisopropylamin und/oder Triethylamin nach allgemein bekannten Verfahren, z. B. in Gegenwart einer Mischung aus N-Ethyldiisopropylamin, Isopropanol, p-Nitrophenol und Dimethylaminopyridin in Pyridin, bei erhöhter Temperatur, z. B. ca. 60 °C, durchgeführt. Die erhaltenen Produkte können anschließend mittels Chromatographie über SiO₂, insbesondere über SiO₂ in Form von Kieselgel, und/oder Kristallisation gereinigt werden.

Die Ausgangsverbindung für das beschriebene erfindungsgemäße Verfahren, das Pentopyranosyl-Nucleosid, läßt sich beispielsweise dadurch herstellen, daß zuerst eine geschützte Nucleobase mit einer geschützten Pentopyranose, bevorzugt einer Ribopyranose umgesetzt wird und anschließend die Schutzgruppen von dem Ribopyranosyl-Teil abgespalten werden. Das Verfahren kann beispielsweise wie bei Pitsch et al. (1993), supra, oder Pitsch et al. (1995), supra, beschrieben durchgeführt werden. Hierbei ist es zur Vermeidung weiterer zeit- und materialaufwendiger Chromatographien vorteilhaft, nur anomerenreine geschützte Pentopyranosen, wie z. B. Tetrabenzoyl-pentopyranosen, vorzugsweise β-Tetrabenzoylribopyranosen (R. Jeanloz, J. Am. Chem. Soc. 1948,70,4052), einzusetzen.

Ein anderer Gegenstand der vorliegenden Erfindung ist daher auch ein Verfahren zur Herstellung eines Ribopyranosyl-nucleosids, bei dem
(a) eine geschützte Nucleobase mit einer geschützten Ribopyranose umgesetzt wird,
(b) die Schutzgruppen von dem Ribopyranosyl-Teil des Produktes aus Schritt (a) abgespalten werden, und
(c) das Produkt aus Schritt (b) gemäß dem oben näher beschriebenen erfindungsgemäßen Verfahren umgesetzt wird.

Zur Herstellung einer Pentopyranosyl-Nucleinsäure wird das erhaltenen Pentopyranosyl-nucleosid in einem weiteren Schritt entweder für die Oligomerisierung phosphityliert oder für die Festphasensynthese an eine feste Phase gebunden. Die Phosphitylierung erfolgt beispielsweise durch Phosphorigsäuremonoallylesterdiisopropyl-amidchlorid in Anwesenheit einer Base, z. B. N-Ethyldiisopropylamin. Die Bindung eines geschützten erfindungsgemäßen Pentopyranosyl-Nucleosids an eine feste Phase, z. B. "long-chain-alkylamino-controlled pore glass" (CPG, Sigma Chemie, München) kann beispielsweise wie bei Pitsch et al. (1993), supra, beschrieben erfolgen.

Ein anderer Gegenstand der vorliegenden Erfindung bezieht sich daher auf ein Verfahren zur Herstellung einer Pentopyranosyl-Nucleinsäure, bei dem
(a) in einem ersten Schritt ein Pentopyranosyl-Nucleosid nach dem oben beschriebenen erfindungsgemäßen Verfahren hergestellt wird,
(b) in einem zweiten Schritt das gemäß Schritt (a) hergestellte Pentopyranosyl-Nucleosid an eine feste Phase gebunden wird, und
(c) in einem weiteren Schritt das gemäß Schritt (b) an eine feste Phase gebundene Pentopyranosylnucleosid um ein phosphityliertes 3'-, 4'-geschütztes Pentopyranosyl-Nucleosid verlängert wird, und
(d) Schritt (c) solange mit gleichen oder verschiedenen phosphitylierten 3'-, 4'geschützten Pentopyranosyl-Nucleosiden wiederholt wird, bis die gewünschte Pentopyranosyl-Nucleinsäure erhalten wird.

In einer besonderen Ausführungsform können in Schritt (b) und/oder Schritt (c) auch die bei der allgemein bekannten Nucleinsäuresynthese üblichen Pentofuranosyl-nucleoside, wie beispielsweise das in ihrer natürlichen Form vorkommende Adenosin, Guanosin, Cytidin, Thymidin und/oder Uracil (siehe z. B. Uhlmann, E. & Peyman, A. (1990). Chemical Reviews, 90, 543-584 No. 4) eingebaut werden, wodurch eine gemischte Nucleinsäure aus Pentopyranosyl-Nucleosiden und Pentofuranosyl-nucleosiden mit neuen Eigenschaften entsteht.

Als Kupplungsreagenz für die Verlängerung gemäß Schritt (c) werden im allgemeinen saure Aktivatoren, wie z.B. einem p-Nitrophenyltetrazol, vorzugsweise 5-(4-Nitrophenyl)-1H-tetrazol, insbesondere Benzimidazoliumtriflat eingesetzt, da bei Benzimidazoliumtriflat im Gegensatz zu 5-(4-Nitrophenyl)-1H-tetrazol als Kupplungsreagenz keine Verstopfung der Kupplungsreagenz-Leitungen und eine Verunreinigung des Produktes erfolgt.

Weiterhin ist es vorteilhaft durch Zusatz von einem Salz, wie Natriumchlorid, zur schutzgruppenabspaltenden Hydrazinolyse die Nucleobasen, insbesondere Pyrimidinbasen, vor allem Uracil und Thymin, vor einer Ringöffnung zu schützen, die das Oligonukleotid zerstören würde. Allyloxygruppen können vorzugsweise durch Palladium [Pd(0)]-Komplexe z. B. vor der Hydrazinolyse abgespalten werden.

Die Abspaltung der gebildeten Nucleinsäure von der festen Phase erfolgt im allgemeinen ebenso durch Hydrazinolyse.

In einer weiteren erfindungsgemäßen Ausführungsform werden daher in einem weiteren Schritt (e) die Schutzgruppen und die gebildete Pentopyranosyl-Nucleinsäure von der festen Phase abgespalten.

Im allgemeinen werden die erfindungsgemäß hergestellten Pentopyranosyl-Nucleinsäuren chromatographisch beispielsweise über alkylsilyliertes Kieselgel, vorzugsweise über RP-C₁₈-Kieselgel, gereinigt.

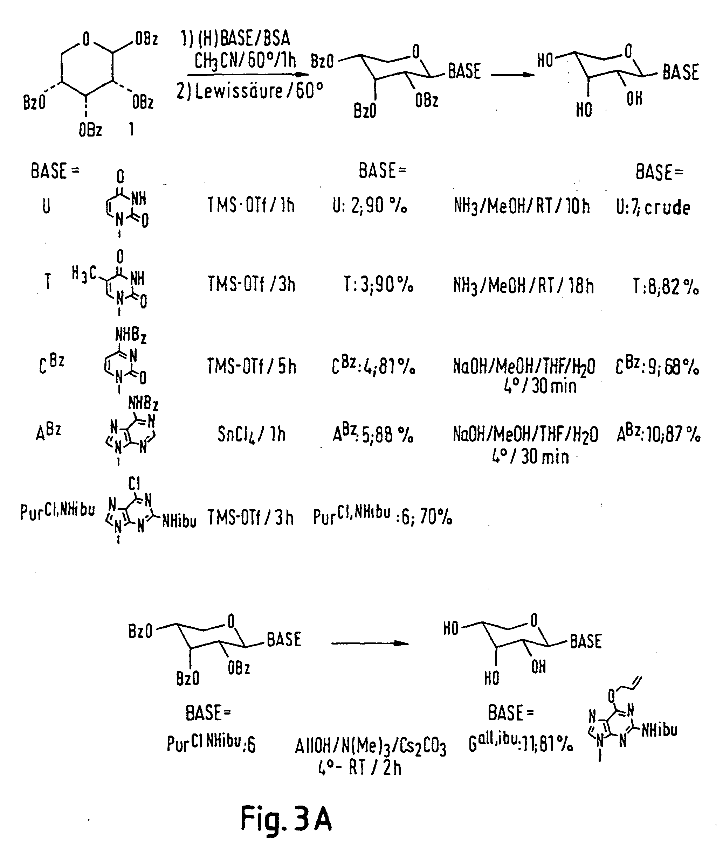

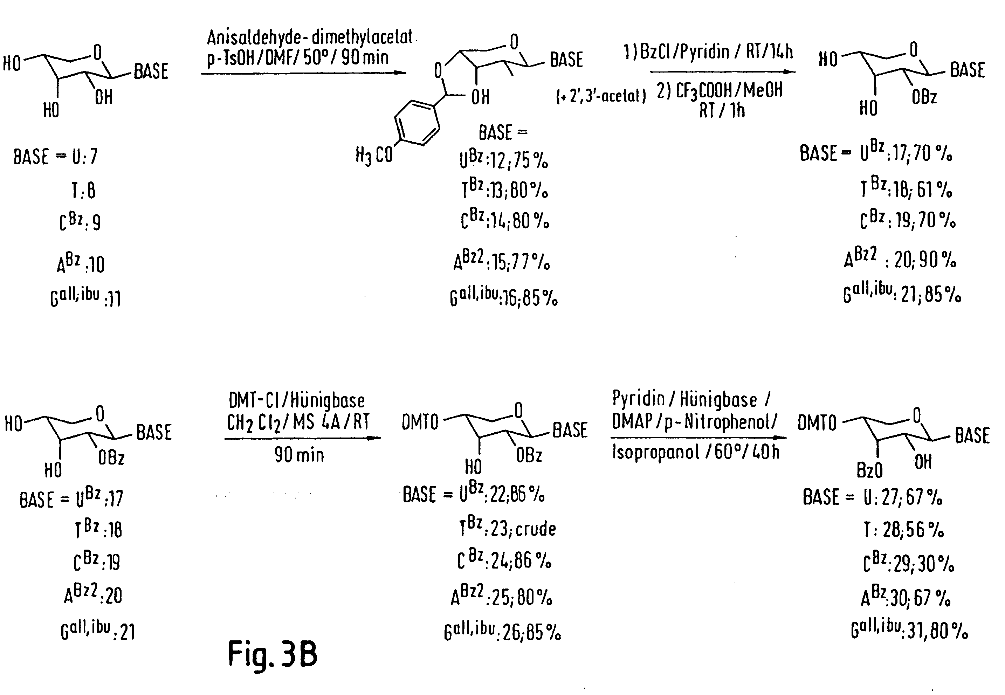

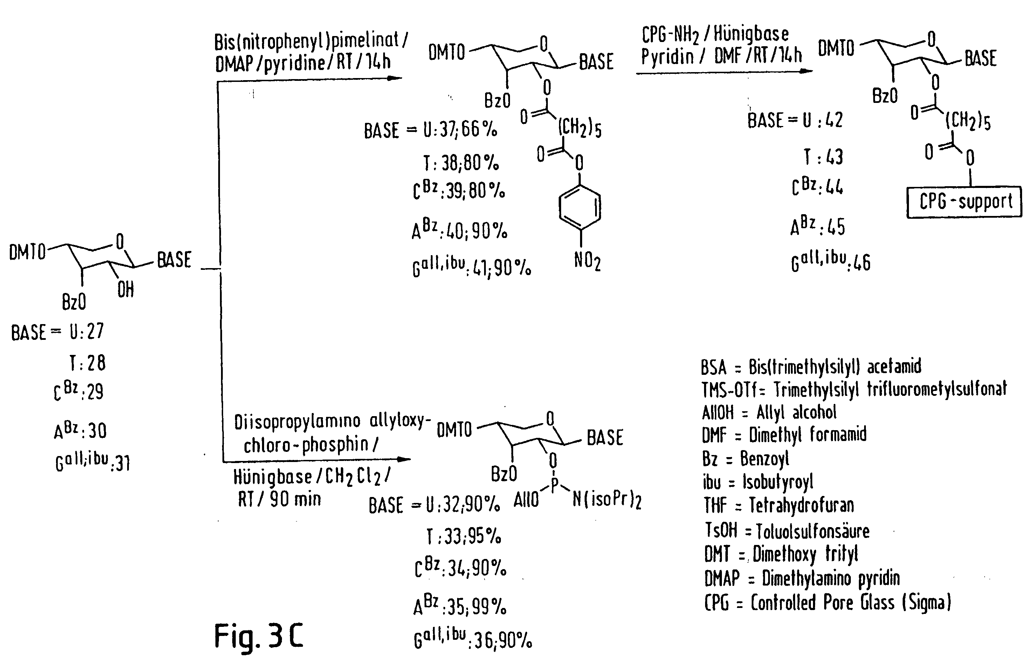

Eine beispielhafte Übersicht über das erfindungsgemäße Verfahren einschließlich weiterer Ausführungsformen liefert Fig. 3.

Die erfindungsgemäß hergestellten Pentopyranosyl-Nucleinsäuren eignen sich beispielsweise zur Herstellung von Paarungssystemen oder Konjugaten.

Paarungssysteme sind supramolekulare Systeme nicht kovalenter Wechselwirkung, die sich durch Selektivität, Stabilität und Reversiblität auszeichnen, und deren Eigenschaften bevorzugt thermodynamisch, d. h. durch Temperatur, pH-Wert und Konzentration beeinflußt werden. Solche Paarungssysteme können z. B. aufgrund ihrer selektiven Eigenschaften auch als "molekularer Klebstoff" für die Zusammenführung von unterschiedlichen Metallclustern zu Cluster-Verbänden, mit potentiell neuen Eigenschaften verwendet werden [siehe z. B. R. L. Letsinger, et al., Nature 1996, 382, 607-9, P. G. Schultz et al., Nature 1996, 382, 609-11]. Folglich eignen sich die p-NA's auch für die Anwendung im Bereich der Nanotechnologie, beispielsweise zur Herstellung neuer Materialien, Diagnostika und Therapeutika sowie mikroelektronischer, photonischer bzw. optoelektronischer Bauteile und für das kontrollierte Zusammenführen molekularer Species zu supramolekularen Einheiten, wie z. B. für den (kombinatorischen) Aufbau von Proteinassemblies [siehe z. B. A. Lombardi, J. W. Bryson, W. F. DeGrado, Biomoleküls (Pept. Sci.) 1997, 40, 495-504], da p-NA's Paarungssysteme bilden, die stark und thermodynamisch kontrollierbar sind. Eine weitere Anwendung ergibt sich daher gerade im diagnostischen und drug discovery-Bereich durch die Möglichkeit, funktionelle, bevorzugt biologische Einheiten wie Proteine oder DNA/RNA-Abschnitte, mit einem p-NA-Code zu versehen, der nicht mit den natürlichen Nucleinsäuren interferiert (siehe z. B. WO93/20242).

Des weiteren kann ein Biomolekül, z. B. DNA oder RNA, zum nicht-kovalenten Verbinden (Linken) mit einem anderen Biomolekül, z. B. DNA oder RNA, verwendet werden, wenn beide Biomoleküle Abschnitte enthalten, die aufgrund komplementärer Sequenzen von Nucleobasen durch Ausbildung von Wasserstoffbrücken aneinander binden können. Derartige Biomoleküle finden z. B. in analytischen Systemen zur Signalamplifizierung Verwendung, wo ein in seiner Sequenz zu analysierendes DNA-Molekül über einen solchen nicht-kovalenten DNA-Linker zum einen an einen festen Träger immobilisiert, und zum anderen an ein signalverstärkendes branchedDNA-Molekül (bDNA) gebunden werden soll (siehe Fig. 3; S. Urdea, Bio/Technol. 1994, 12, 926 oder US-Patent Nr. 5,624,802). Ein wesentlicher Nachteil der zuletzt beschriebenen Systeme ist, daß sie den Verfahren zur Nucleinsäure-Diagnostik durch Polymerase-Chain-Reaction (PCR) (K. Mullis, Methods Enzymol. 1987, 155, 335) hinsichtlich der Empfindlichkeit bis jetzt unterlegen sind. Das ist u. a. darauf zurückzuführen, daß die nicht-kovalente Bindung vom festen Träger an das zu analysierende DNA-Molekül ebenso wie die nicht-kovalente Bindung des zu analysierenden DNA-Moleküls nicht immer spezifisch erfolgt, wodurch es zu einer Vermischung der Funktionen "Sequenzerkennung" und "nicht-kovalente Bindung" kommt. Die Verwendung von p-NA's als orthogonales Paarungssystem, welches nicht in das DNA- bzw. RNA-Paarungsgeschehen eingreift, löst dieses Problem auf vorteilhafte Weise, wodurch die Empfindlichkeit der beschriebenen analytischen Verfahren deutlich erhöht werden kann.

Konjugate sind im Sinne der vorliegenden Erfindung kovalent gebundene Hybride aus p-NA's und anderen Biomolekülen, vorzugsweise ein Peptid, Protein oder eine Nucleinsäure, beispielsweise ein Antikörper oder ein funktioneller Teil davon oder eine in ihrer natürlichen Form vorkommende DNA und/oder RNA. Funktionelle Teile von Antikörper sind beispielsweise Fv-Fragmente (Skerra & Plückthun (1988) Science 240, 1038), einzelkettige Fv-Fragmente (scFv; Bird et al. (1988), Science 242, 423, Huston et al. (1988) Proc. Natl. Acad. Sci. U.S.A., 85, 5879) oder Fab-Fragmente (Better et al. (1988) Science 240, 1041). Bevorzugt sind im allgemeinen p-RNA/DNA- bzw p-RNA/RNA-Konjugate.

Konjugate werden dann vorzugsweise verwendet, wenn die Funktionen "Sequenzerkennung" und "nicht-kovalente Bindung" in einem Molekül realisiert werden müssen, da die Konjugate zwei zueinander orthogonale Paarungssysteme enthalten.

Unter dem Begriff Konjugat im Sinne der vorliegenden Erfindung sind auch sogenannte Arrays zu verstehen. Arrays sind Anordnungen von immobilisierten Erkennungsspecies, die speziell in der Analytik und Diagnostik eine wichtige Rolle bei der simultanen Bestimmung von Analyten spielen. Beispiele sind Peptide-Arrays (Fodor et al., Nature 1993, 364, 555) und Nucleinsäure-Arrays (Southem et al. Genomics 1992, 13, 1008; Heller, US-Patent Nr. 5,632,957). Eine höhere Flexibilität dieser Arrays kann dadurch erreicht werden, daß die Erkennungsspecies an codierende Oligonucleotide gebunden werden und die zugehörigen, komplementären Stränge an bestimmte Positionen auf einem festen Träger. Durch Aufbringen der codierten Erkennungsspecies auf den "anti-codierten" festen Träger und Einstellung von Hybridisierungsbedingungen werden die Erkennungsspecies an den gewünschten Positionen nicht-kovalent gebunden. Dadurch können verschiedene Typen von Erkennungsspecies, wie z. B. DNA-Abschnitte, Antikörper, nur durch Anwendung von Hybridisierungsbedingungen gleichzeitig auf einem festen Träger angeordnet werden. Voraussetzung hierzu sind aber äußerst starke, selektive - um die codierenden Abschnitte möglichst kurz zu halten - und mit natürlicher Nucleinsäure nicht interferierender Codons und Anticodons notwendig. p-NA's, vorzugsweise p-RNA's eignen sich hierzu in besonders vorteilhafter Weise.

Für die Herstellung von Konjugaten sind sowohl sequentielle als auch konvergente Verfahren geeignet, wobei sich konvergente Verfahren aufgrund ihrer Flexibilität als besonders bevorzugt erweisen.

In einem sequentiellen Verfahren wird z. B. nach erfolgter automatisierter Synthese eines p-RNA-Oligomeren direkt am gleichen Synthesizer - nach Umstellung der Reagenzien und des Kupplungsprotokolls - z. B. ein DNA-Oligonukleotid weitersynthetisiert. Dieser Vorgang läßt sich auch in umgekehrter Reihenfolge durchführen.

In einem konvergenten Verfahren werden z. B. p-RNA-Oligomere mit Aminoterminalen-Linkern und z. B. DNA-Oligomere mit z. B. Thiol-Linkern in getrennten Vorgängen synthetisiert. Anschließend erfolgt vorzugsweise eine Jodacetylierung des p-RNA-Oligomeren und die Kupplung der beiden Einheiten nach literaturbekannten Protokollen (T. Zhu et al., Bioconjug. Chem. 1994, 5, 312).

Besonders bevorzugte aminoterminale Linker sind Allyloxy-Linker der Formel (IV)

S_{c1}NH(CₙH₂ₙ)CH(OPS_{c2}S_{c3})CₙH₂ₙS_{c4} (IV),

worin S_{c1} und S_{c4} unabhängig voneinander, gleich oder verschieden, jeweils eine Schutzgruppe ausgewählt aus Fmoc und/oder DMT,
S_{c2} und S_{c3} unabhängig voneinander, gleich oder verschieden, jeweils eine Allyloxy- und/oder Diisopropylamino-Gruppe und n eine ganze Zahl von 1-12, vorzugsweise 1-8, insbesondere 1-4 bedeuten. Ein besonders bevorzugter Allyloxy-Linker ist 2-(S)-N-Fmoc-O¹-DMT-O²-allyloxydiisopropylaminophosphinyl-6-amino-1,2-hexandiol.

2-(S)-N-Fmoc-O¹-DMT-O²-allyloxydiisopropylaminophosphinyl-6-amino-1,2-hexandiol läßt sich beispielsweise aus 6-Amino-2(S)-hydroxyhexansäure herstellen. 6-Amino-2(S)-hydroxyhexansäure kann nach literaturbekannter Weise durch Diazotierung und anschließender Hydrolyse aus L-Lysin hergestellt werden (K.-I. Aketa, *Chem. Pharm Bull*., 24, 621 (1976)). Dieses wird anschließend mit FmocCl zum 2-(S)-N-Fmoc-6-amino-1,2-hexandiol umgesetzt, welches gemäß WO 89/02439 zum (2-(S)-N-Fmoc-O¹-DMT-6-amino-1,2-hexandiol DM-trityliert werden kann. Dieses wird beispielsweise in Anwesenheit von Ethyldiisopropylamin und Chlor-N,N-diisopropylaminoallyloxyphosphin zum 2-(S)-N-Fmoc-O¹-DMT-O²-allyloxydiisopropylaminophosphinyl-6-amino-1,2-hexandiol umgesetzt.

Ausgehend von z. B. Lysin können somit in wenigen Reaktionsschritten aminoterminale Linker aufgebaut werden, die sowohl eine aktivierbare Phosphorverbindung als auch eine säurelabile Schutzgruppe, wie DMT, tragen und daher leicht in der automatisierbaren Oligonucleotidsynthese verwendet werden können (siehe z. B. P. S. Nelson et al., *Nucleic Acid Res.* 17, 7179 (1989); L. J. Amold et al., WO 89/02439). Ein Lysin-basierender Linker, bei dem anstelle der sonst üblichen Cyanoethyl-Gruppe am Phosphoratom eine Allyloxy-Gruppe eingebracht ist, kann in vorteilhafter Weise in der Noyori-Oligonucleotid-Methode eingesetzt werden (R. N oyori, *J. Am. Chem. Soc*. 112, 1691-6 (1990)).

In einer weiteren Ausführungsform des erfindungsgemäßen Verfahrens zur Herstellung von Pentopyranosyl-Nucleinsäuren wird daher in einem weiteren Schritt ein Allyloxy-Linker der Formel (III)

S_{c1}NH(CₙH₂ₙ)CH(OPS_{c2}S_{c3})CₙH₂ₙS_{c4} (IV),

worin S_{c1} und S_{c4} unabhängig voneinander, gleich oder verschieden, jeweils eine Schutzgruppe ausgewählt aus Fmoc und/oder DMT,
S_{c2} und S_{c3} unabhängig voneinander, gleich oder verschieden, jeweils eine Allyloxy- und/oder Diisopropylamino-Gruppe und n eine ganze Zahl von 1-12, vorzugsweise 1-8, insbesondere 1-4 bedeuten, eingebaut.

Daneben weisen Indolderivate als Linker (s. z.B. Formel (I) in Verbindung mit Formel (III)) den Vorzug der Fluoreszenzfähigkeit auf und sind daher für Nanotechnologie-Anwendungen, bei denen es ggf. um den Nachweis kleinster Substanzmengen geht, besonders bevorzugt. Beispielsweise eignen sich Indol-1-riboside, wie bei N. N. Suvorov et al., *Biol. Aktivn. Soedin*., Akad. Nauk SSSR, 60 (1965) und Tetrahedron 23, 4653 (1967) bereits beschrieben. 3-substituierte Derivate werden im allgemeinen über die Bildung eines Aminals der ungeschützten Zuckerkomponente und einem Indolin, welches dann durch Oxidation in das Indol-1-ribosid übergeführt wird, hergestellt. Beschrieben wurden z. B. Indol-1-glucoside und -1-arabinoside (Y. V. Dobriynin et al., *Khim.-Farm. Zh*. 12, 33 (1978)), deren 3-substituierte Derivate im allgemeinen über Vielsmeier-Reaktion hergestellt werden können.

Für die Herstellung Indol-basierender Linker geht man beispielsweise von Phthalsäureanhydrid und Tryptamin aus, welche zum N-Phthaloyltryptamin umgesetzt werden (Kuehne et al., *J. Org. Chem*. 43, 13, 2733-2735 (1987)). Dieses wird z. B. mit Boran-THF zum Indolin reduziert (analog A. Giannis et al., *Angew. Chem.* 101, 220 (1989)). Anschließend kann das 3-substituierte Indolin zuerst mit Ribose zum Nucleosidtriol und dann mit Essigsäureanhydrid zum Triacetat umgesetzt werden. Anschließend oxidiert man beispielsweise mit 2,3-Dichlor-5,6-dicyanoparachinon, spaltet die Acetate mit z. B. Natriummethylat, benzoyliert selektiv in 2'-Position, DM-trityliert selektiv in 4'-Position, und führt die Wanderungsreaktion zum 3'-Benzoat durch. Die Bildung des Phosphoramidits erfolgt nach bekannten Verfahren Dieser läßt sich ohne Änderung der Syntheseprotokolle für die automatisierte Oligonucleotidsynthese einsetzen.

Weitere für das erfindungsgemäße Verfahren geeignete Linker (s. z.B. Formel (II) in Verbindung mit Formle (III)) sind Uracil-basierende Linker, bei denen die 5'-Position des Uracils modifiziert wurde. Ein geeignetes Beispiel ist N-Phthaloylaminoethyluracil, das aus Hydroxyethyluracil gewonnen werden kann.

Die Herstellung von Hydroxyethyluracil gelingt im großen Maßstab nach bekannter Methode (J.D. Fissekis, A. Myles, G.B. Brown, *J. Org. Chem*. 29, 2670 (1964)). Anschließend wird beispielsweise das erhaltene Hydroxyethyluracil mit Methansulfonsäurechlorid in Pyridin mesyliert zu (J.D. Fissekis, F. Sweet, *J. Org.* *Chem*. 38, 264 (1973)). Danach wird im allgemeinen mit Natriumazid in DMF das Reaktionsprodukt zum Azid umgesetzt und dieses mit Triphenylphosphin in Pyridin zum Aminoethyluracil reduziert. Die Aminofunktion wird schließlich z.B. mit N-Ethoxycarbonylphthalimid geschützt. Nukleosidierung eines Ribosetetrabenzoats mit N-Phthaloylaminoethyluracil liefert beispielsweise einen Ribosetribenzoat-Linker in guten Ausbeuten. Anschließende Abspaltung der Benzoatschutzgruppen mit NaOMe in MeOH liefert das Linkertriol, welches beispielsweise bei -78°C in Pyridin/Dichlormethan 1:10 in Gegenwart von DMAP mit Benzoylchlorid umgesetzt werden kann. Dabei erhält man neben dem gewünschten 2'-Benzoat (64%) auch 2',4'-dibenzoyliertes Produkt (22%), welches gesammelt und wieder in das Triol umgewandelt werden kann. Das 2'-Benzoat wird z.B. mit Dimethoxytritylchlorid in Gegenwart von Hünig-Base in Dichlormethan in 4'-Position in Ausbeuten größer 90% trityliert. Die Umlagerung von 4'-DMT-2'-benzoat zum 4'-DMT-3'-benzoat erfolgt z. B. in Gegenwart von DMAP, p-Nitrophenol und Hünig-Base in n-Propanol/Pyridin 5:2. Nach Chromatographie wird das 4'-DMT-3'-benzoat erhalten, welches abschließend z. B. mit CIP(OAII)N(iPr)₂ in Gegenwart von Hünig-Base zum Phosphoramidit umgesetzt werden kann. Dieser läßt sich ohne Änderung der Syntheseprotokolle für die automatisierte Oligonucleotidsynthese einsetzen.

Die folgenden Figuren und Beispiele sollen die Erfindung näher beschreiben, ohne sie zu beschränken.

### BESCHREIBUNG DER FIGUREN

- Fig. 1: zeigt einen Ausschnitt aus der Struktur von RNA in ihrer natürlich vorkommenden Form (links) und in Form einer p-NA (rechts).
- Fig. 2: zeigt schematisch die Synthese eines p-Ribo(A,U)-Oligonucleotids nach Pitsch et al. (1993).
- Fig. 3: zeigt schematisch das erfindungsgemäße Verfahren einschließlich weiterer Ausführungsformen.

### DEFINITIONEN DER ABKÜRZUNGEN

- BSA: bedeutet Bis(trimethylsilyl)acetamid
- TMS-OTf: bedeutet Trimethylsilyltrifluoromethylsulfonat
- AIIOH: bedeutet Allylalkohol
- DMF: bedeutet Dimethylformamid
- Bz: bedeutet Benzoyl
- ibu: bedeutet Isobutyryl
- THF: bedeutet Tetrahydrofuran
- TsOH: bedeutet Toluolsulfonsäure
- DMT: bedeutet Dimethoxytrityl
- DMAP: bedeutet Dimethylaminopyridin
- CPG: bedeutet "controlled pore glass"

### BEISPIELE

### Synthese eines Oligomeren der Sequenz G₃CG₃C

### Vorbereitung:

Die für die Synthese der geplanten Sequenz benötigte Menge an Phosphoramiditen (320 µl / Kopplungsschritt) sowie das benötigte Quantum an Tetrazol-Gemisch (650 µl Kopplungsschritt einer 0,35 M Tetrazol / 0,15 M p-Nitrophenyltetrazol-Lsg.) wurde je in ein Synthesizer-Gläschen eingewogen und mindestens 14 Stunden im Hochvakuum über KOH-Plätzchen (Exsikkator) getrocknet. Dann wurde die Mischung in dem benötigten Volumen an CH₃CN gelöst und mit ca. 5 Kugeln aktiviertem Molekularsieb 4 Å versetzt. Die Gläschen wurden mit einem Septum verschlossen und mindestens weitere 14 Stunden bei RT aufbewahrt.

### Aufbau:

Der Aufbau der Sequenz am DNA-Synthesizer (Pharmacia Gene Assembler) erfolgte prinzipiell wie derjenige von DNA-Oligonukleotiden gem‰fl den Standardbedingungen des Automatenherstellers PHARMACIA, D-Freiburg. Bei der Synthese wurde die letzte Tritylgruppe am Oligonukleotid belassen ("Trityl on"). Es wurden folgende Änderungen der Standardbedingungen eingeführt:
1. Die Detritylierungszeiten wurden auf 7 min. verlängert;
2. Es wurde eine 6 %ige (statt 3 %ige) Lösung von Dichloressigsäure in Dichlorethan verwendet;
3. Die Kopplungszeit wurde auf 30 min. verlängert.

Für den Aufbau wurde von 400 mg eines mit p-Ribo-C-Baustein beladenen Trägers ausgegangen (= 10 µmol).

### Entschützung:

Nach dem Aufbau wurde der sich noch in der Kartusche befindende Träger am Vakuum getrocknet (ca. 30 min.) und mit einer vorbereiteten Lösung von 60 mg (66 µmol) Tetrakis(triphenylphosphin)-palladium(0), 60 mg Triphenylphosphin (225 µmol), und 60 mg (170 µmol) Diethylammonium-hydrogencarbonat in 4,5 ml CH₂Cl₂ versetzt. Das Gemisch wurde 5 Stunden bei Raumtemperatur (RT) geschüttelt, dann der Träger abfiltriert und nacheinander mit 20 ml CH₂Cl₂ und 25 ml Aceton gewaschen. Er wurde in 4,5 ml 0,1 M Natrium-N,N-diethyldithiocarbamat-Lösung aufgenommen und 30 min. bei RT stehengelassen. Danach wurde wiederum abfiltriert und es wurde nacheinander mit 10 ml H₂O, 15 ml Aceton und 10 ml EtOH gewaschen.

Anschließend wurde der Träger in 3,6 ml H₂O / 0,9 ml Hydrazin-Hydrat aufgenommen und 30 Stunden bei 4° (Kühlraum) mittels eines Motors gedreht. Dann wurde an 1 x 4 cm RP-C₁₈-Kieselgel chromatographiert. Dazu wurde das Säulenmaterial in CH₃CN aufgeschlämmt und in eine Säule (1 x 5 cm) gefüllt. Es wurde mit 50 ml CH₃CN, 50 ml 2 % NEt₃ in CH₃CN und dann mit 50 ml 0,1 M TEAB-Puffer konditioniert. Daraufhin wurde die Probe in 0,1 M TEAB-Puffer aufgetragen; es wurde mit 50 ml 0,1 M TEAB-Puffer, 30 ml H₂O und dann mit H₂O → H₂O/CH₃CN 1 : 1 eluiert. Die Detektion der Produkte erfolgte UV-photometrisch. Die produkthaltigen Fraktionen wurden eingedampft, in 15 ml H₂O/HCOOH 1 : 4 aufgenommen, 15 min. bei RT stehengelassen, eingedampft, mit 5 ml H₂O versetzt, wiederum eingedampft und an Sepak (Fa. Waters) chromatographiert. Dazu wurde die Kartusche mit 15 ml CH₃CN und dann mit 15 ml 0,1 M TEAB-Puffer gewaschen. Die Oligonukleotid-haltige Lösung wurde in 0, 1 M TEAB-Puffer aufgetragen; dann wurde mit 10 ml 0,1 M TEAB-Puffer und anschließend mit einem H₂O/CH₃CN-Gradienten eluiert (0 → 50 %). Es wurden Fraktionen à 1,5 ml gesammelt; diese wurden im UV analysiert.

Die produkthaltigen Fraktionen wurden eingedampft und HPLC-chromatographisch gereinigt. Danach wurden die vereinigten, produkthaltigen Fraktionen an Sepak, wie oben beschrieben, entsalzt. Das Produkt wurde als wäßrige Lösung (10 ml) im gefrorenen Zustand aufbewahrt. UV-spektroskopisch ergab sich eine Ausbeute von 25 % (= 250 o.D.)

### Charakterisierung:

HPLC: Retentionszeit 15,3 min. an einer *Aquapore* RP-300, 7 µm, 220 x 4,6 mm, Fluß 1 ml / min.; Puffer A: 0,1 M NEt₃ / 0,1 M AcOH (pH 7,0) in H₂O, Puffer B: 0,1 M NEt₃ / 0,1 M AcOH (pH 7,0) in H₂O / CH₃CN 1 : 4; Gradient: 100% A → 70% A / 30 % B. Detektion 260 nm.
UV: λₘₐₓ: 257 nm.
MALDI-TOF-MS: [M-1]_{calc}= 2617; [M-1]_{obs}=2617.

## Patentansprüche

1. Verfahren zur Herstellung eines 3', 4'-cyclischen Acetals eines Pentopyranosyl-Nucleosids, **dadurch gekennzeichnet, daß** ein Pentopyranosyl-Nucleosid mit einem Aldehyd, Keton, Acetal oder Ketal unter Unterdruck bei weniger als 500 mbar umgesetzt wird.

2. Verfahren nach Anspruch 1, **dadurch gekennzeichnet, daß** der Aldehyd ausgewählt ist aus Formaldehyd, Acetaldehyd, Benzaldehyd oder 4-Methoxybenzaldehyd, das Acetal aus Formaldehyd-dimethylacetal, Acetaldehyddimethylacetal, Benzaldehyd-dimethylacetal, oder 4-Methoxybenzaldehyd-dimethylacetal, das Keton aus Aceton, Cyclopentanon oder Cyclcohexanon und das Ketal aus Acetondimethylketal, Cyclopentanon-dimethylketal, Cyclohexanon-dimethylketal oder in Form von 2-Methoxypropen.

3. Verfahren nach der Anspruch 1 oder 2, **dadurch gekennzeichnet, daß** das Pentopyranosyl-Nucleosid vor der Umsetzung gereinigt wird.

4. Verfahren nach Anspruch 3, **dadurch gekennzeichnet, daß** das Pentopyranosyl-Nucleosid über SiO₂ gereinigt wird.

5. Verfahren nach einem der Ansprüche 1-4, **dadurch gekennzeichnet, daß** als Pentopyranosyl-Nucleosid ein Ribo-, Arabino-, Lyxo- oder Xylo-pyranosyl-Nucleosid eingesetzt wird.

6. Verfahren nach einem der Ansprüche 1-5, **dadurch gekennzeichnet, daß** als Pentopyranosyl-Nucleosid ein Pentopyranosyl-purin, -2,6-diaminopurin, -6-purinthiol, -pyridin, -pyrimidin, -adenosin, -guanosin, -isoguanosin, -6-thioguanosin, -xanthin, -hypoxanthin, -thymidin, -cytosin, -isocytosin , -indol, -tryptamin, -N-phthaloyltryptamin, -uracil, -coffein, -theobromin, -theophyllin, -benzotriazol oder -acridin eingesetzt wird.

7. Verfahren nach einem der Ansprüche 1-6, **dadurch gekennzeichnet, daß** ein Pentopyranosyl-Nucteosid der Formel (I) 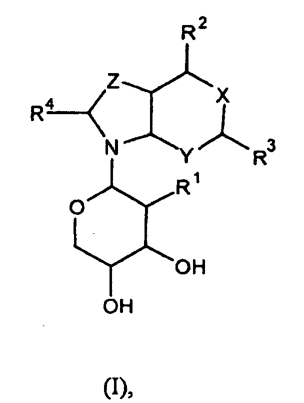 worin
R¹ gleich H, OH oder Hal mit Hal gleich Br oder Cl,
R², R³ und R⁴ unabhängig voneinander, gleich oder verschieden, jeweils H, Hal mit Hal gleich Br oder Cl, NR⁵R⁶, OR⁷, SR⁸, =O, CₙH₂ₙ₊₁ mit n eine ganze Zahl von 1-12 oder (CₙH₂ₙ)NR¹⁰R¹¹ mit R¹⁰R¹¹ gleich H, CₙH₂ₙ₊₁ oder R¹⁰R¹¹ verbunden über einen Rest der Formel 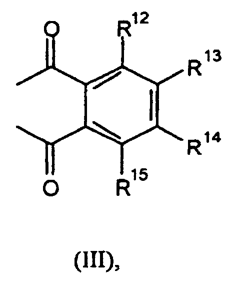 worin R¹², R¹³, R¹⁴ und R¹⁵ unabhängig voneinander, gleich oder verschieden, jeweils H, OR⁷, wobei R⁷ die genannte Bedeutung hat, oder CₙH₂ₙ₊₁, oder CₙH₂ₙ₋₁, wobei n die oben genannte Bedeutung hat, bedeuten und
R⁵, R⁶, R⁷ und R⁸ unabhängig voneinander, gleich oder verschieden, jeweils H, CₙH₂ₙ₊₁, oder CₙH₂ₙ₋₁, wobei n die oben genannte Bedeutung hat, -C(O)R⁹ mit R⁹ gleich ein linearer oder verzweigter, gegebenenfalls substituierter Alkyl-, Aryl-, vorzugsweise Phenyl-Rest,
X, Y und Z unabhängig voneinander, gleich oder verschieden, jeweils =N-, =C(R¹⁶)- oder -N(R¹⁷)- mit R¹⁶ und R¹⁷ unabhängig voneinander, gleich oder verschieden, jeweils H oder CₙH₂ₙ₊₁ oder (CₙH₂ₙ)NR¹⁰R¹¹ mit den oben genannten Bedeutungen, bedeutet,
oder der Formel (II) 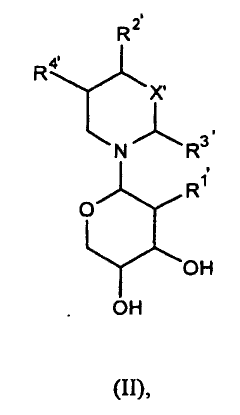 worin R^{1'} gleich H, OH oder Hal mit Hal gleich Br oder Cl,
R^{2'}, R^{3'} und R^{4'} unabhängig voneinander, gleich oder verschieden, jeweils H, Hal mit Hal gleich Br oder Cl, =O, CₙH₂ₙ₊₁ oder OCₙH₂ₙ₋₁, oder (CₙH₂ₙ)NR^{10'}R^{11'}, wobei R^{10'}, R^{11'}, unabhängig voneinander die oben genannte Bedeutung von R¹⁰ bzw. R¹¹ hat, und
X' jeweils =N-, =C(R^{16'})- oder -N(R^{17'})- bedeutet, wobei R^{16'} und R^{17'} unabhängig voneinander die oben genannte Bedeutung von R¹⁶ bzw. R¹⁷ haben, umgesetzt wird.

8. Verfahren nach einem der Ansprüche 1-7, **dadurch gekennzeichnet, daß** die Umsetzung bei einer Temperatur von 40-70 °C erfolgt.

9. Verfahren nach einem der Ansprüche 1-8, **dadurch gekennzeichnet, daß** die Umsetzung in Gegenwart einer Säure erfolgt.

10. Verfahren nach Anspruch 9, **dadurch gekennzeichnet, daß** die Säure ausgewählt ist aus p-Toluolsulfonsäure, Methansulfonsäure, Tetrafluoroborsäure, Schwefelsäure, sauren Ionenaustauscher und/oder Lewissäuren.

11. Verfahren nach einem der Ansprüche 1-10, **dadurch gekennzeichnet, daß** in einem weiteren Schritt das erhaltene 3', 4'-cyclische Acetal eines Pentopyranosyl-Nucleosids an der 2'-Position geschützt wird.

12. Verfahren nach Anspruch 11, **dadurch gekennzeichnet, daß** die 2'-Position durch eine basenlabile oder metallkatalysiert abspaltbare Schutzgruppe, geschützt wird.

13. Verfahren nach Anspruch 11 oder 12, **dadurch gekennzeichnet, daß** die 2'-Position durch eine Acylgruppe, Acetyl-, Benzoyl-, Nitrobenzoyl- und/oder Methoxybenzoylgruppe geschützt wird.

14. Verfahren zur Herstellung eines Pentopyranosyl-Nucleosids nach einem der Ansprüche 11-13, **dadurch gekennzeichnet, daß** das an der 2'-Position geschützte 3', 4'-cyclische Acetal eines Pentopyranosyl-Nucleosids deketalisiert wird.

15. Verfahren nach Anspruch 14, **dadurch gekennzeichnet, daß** die Deketalisierung in Gegenwart einer Säure, vorzugsweise in Gegenwart einer starken Säure erfolgt.

16. Verfahren nach Anspruch 14 oder 15, **dadurch gekennzeichnet, daß** das erhaltene Reaktionsprodukt trocken-basisch aufgearbeitet wird.

17. Verfahren nach Anspruch 16, **dadurch gekennzeichnet, daß** die trockenbasische Aufarbeitung in Anwesenheit von festem Hydrogencarbonat, Carbonat und/oder basischem Ionenaustauscher erfolgt.

18. Verfahren nach einem der Ansprüche 14-17, **dadurch gekennzeichnet, daß** in einem weiteren Schritt die 4'-Position geschützt wird.

19. Verfahren nach Anspruch 18, **dadurch gekennzeichnet, daß** die 4'-Position durch eine säure- oder basenlabile Schutzgruppe geschützt wird.

20. Verfahren nach Anspruch 19, **dadurch gekennzeichnet, daß** die 4'-Position durch eine Trityl-Gruppe, eine DMT-Gruppe, eine β-eliminierbare Gruppe oder eine Fmoc-Gruppe geschützt wird.

21. Verfahren nach einem der Ansprüche 18-20, **dadurch gekennzeichnet, daß** in einem weiteren Schritt eine Umlagerung der Schutzgruppe von der 2'-Position zur 3'-Position erfolgt.

22. Verfahren nach Anspruch 21, **dadurch gekennzeichnet, daß** die Umlagerung in Gegenwart einer Base durchgeführt wird.

23. Verfahren nach Anspruch 22, **dadurch gekennzeichnet, dass** die Umlagerung in Gegenwart von N-Ethyldiisopropylamin und/oder Triethylamin durchgeführt wird.

24. Verfahren zur Herstellung eines Pentopyranosyl-nucleosids, **dadurch gekennzeichnet, daß**
(a) eine geschützte Nucleobase mit einer geschützten Pentopyranose umgesetzt wird,
(b) die Schutzgruppen von dem Pentopyranosyl-Teil des Produktes aus Schritt (a) abgespalten werden, und
(c) das Produkt aus Schritt (b) gemäß dem Verfahren nach einem der Ansprüche 1-23 umgesetzt wird.

25. Verfahren nach einem der Ansprüche 21-24, **dadurch gekennzeichnet, daß** das erhaltene Pentopyranosyl-nucleosid in einem weiteren Schritt phosphityliert oder an eine feste Phase gebunden wird.

26. Verfahren zur Herstellung einer Pentopyranosyl-Nucleinsäure, **dadurch gekennzeichnet, daß**
(a) in einem ersten Schritt ein Pentopyranosyl-Nucleosid nach einem der Ansprüche 21-24 hergestellt wird,
(b) in einem zweiten Schritt das gemäß Schritt (a) hergestellte Pentopyranosyl-Nucleosid an eine feste Phase gebunden wird, und
(c) in einem weiteren Schritt das gemäß Schritt (b) an eine feste Phase gebundene 3'-, 4'-geschützte Pentopyranosylnucleosid um ein phosphityliertes 3'-, 4'-geschütztes Pentopyranosyl-Nucleosid verlängert wird, und
(d) Schritt (c) wiederholt wird.

27. Verfahren nach Anspruch 26, **dadurch gekennzeichnet, daß** in Schritt (b) und/oder Schritt (c) auch mindestens ein Pentofuranosyl-nucleosid eingebaut wird.

28. Verfahren nach Anspruch 26 oder 27, **dadurch gekennzeichnet, daß** als Kupplungsreagenz für die Verlängerung gemäß Schritt (c) saure Aktivatoren eingesetzt werden.

29. Verfahren nch Anspruch 28, **dadurch gekennzeichnet, dass** als saure Aktivatoren p-Nitrophenyltetrazol oder Benzimidazoliumtriflat eingesetzt werden.

30. Verfahren nach einem der Ansprüche 26-29, **dadurch gekennzeichnet, daß** in einem weiteren Schritt (e) die Schutzgruppen und das gebildete Oligomer von der festen Phase abgespalten werden.

31. Verfahren nach Anspruch 30, **dadurch gekennzeichnet, daß** die Abspaltung durch Hydrazinolyse erfolgt.

32. Verfahren nach Anspruch 30 oder 31, **dadurch gekennzeichnet, daß** das erhaltene Oligomer chromatographisch gereinigt wird.

33. Verfahren nach Anspruch 32, **dadurch gekennzeichnet, daß** die chromatographische Reinigung über alkylsilyliertem Kieselgel erfolgt.

34. Verfahren nach einem der Ansprüche 28-33, **dadurch gekennzeichnet, daß** in einem weiteren Schritt ein Allyloxy-Linker der Formel (III)
S_{c1}NH(CₙH₂ₙ)CH(OPS_{c2}S_{c3})CₙH₂ₙS_{c4} (III),
worin S_{c1} und S_{c4} unabhängig voneinander, gleich oder verschieden, jeweils eine Schutzgruppe ausgewählt aus Fmoc und/oder DMT,
S_{c2} und S_{c3} unabhängig voneinander, gleich oder verschieden, jeweils eine Allyloxy- und/oder Diisopropylamino-Gruppe und n eine ganze Zahl von 1-12, vorzugsweise 1-8, insbesondere 1-4 bedeuten, eingebaut wird.

35. Verfahren nach Anspruch 34, **dadurch gekennzeichnet, dass** als Allyloxylinker 2-(S)-N-Fmoc-O¹-DMT-O²-allyloxydiisopropylaminophosphinyl-6-amino-1,2-hexandiol eingebaut wird.

## Claims

1. A process for the preparation of a 3',4'-cyclic acetal of a pentopyranosylnucleoside, wherein the pentopyranosylnucleoside is reacted with an aldehyde, ketone, acetal or ketal under reduced pressure at less than about 500 mbar.

2. The process as claimed in claim 1, wherein the aldehyde is selected from formaldehyde, acetaldehyde, benzaldehyde or 4-methoxybenzaldehyde, the acetal is selected from formaldehyde dimethyl acetal, acetaldehyde dimethyl acetal, benzaldehyde dimethyl acetal, or 4-methoxybenzaldehyde dimethyl acetal, the ketone is selected from acetone, cyclopentanone or cyclohexanone and the ketal is selected from acetone dimethyl ketal, cyclopentanone dimethyl ketal, cyclohexanone dimethyl ketal or is in the form of 2-methoxypropene.

3. The process as claimed in claim 1 or 2, wherein the pentopyranosylnucleoside is purified before the reaction.

4. The process as claimed in one of claims 3, wherein the pentopyranosylnucleoside is purified over SiO₂.

5. The process as claimed in one of claims 1-4, wherein the pentopyranosylnucleoside employed is a ribo-, arabino-, lyxo- or xylopyranosylnucleoside.

6. The process as claimed in one of claims 1-5, wherein the pentopyranosylnucleoside employed is a pentopyranosylpurine, -2,6-diaminopurine, -6-purinethiol, -pyridine, - pyrimidine, -adenosine, -guanosine, -isoguanosine, -6-thioguanosine, -xanthine, -hypoxanthine, -thymidine, -cytosine, -isocytosine, -indole, -tryptamine, -N-phthaloyltryptamine, -uracil, -coffeine, -theobromine, -theophylline, -benzotriazole or -acridine.

7. The process as claimed in one of claims 1-6, wherein a pentopyranosylnucleoside of the formula (I) 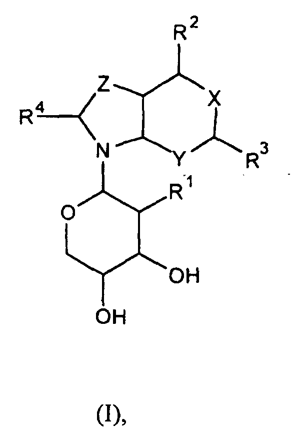 in which
R¹ is equal to H, OH or Hal where Hal is equal to Br or Cl,
R², R³ and R⁴ independently of one another, identically or differently, are each H, Hal where Hal is equal to Br or Cl, NR⁵R⁶, OR⁷, SR⁸, =O, CₙH₂ₙ₊₁ where n is an integer from 1-12, or (CₙH₂ₙ)NR¹⁰R¹¹ where R¹⁰, R¹¹ are equal to H, CₙH₂ₙ₊₁ or R¹⁰R¹¹ is a radical of the formula 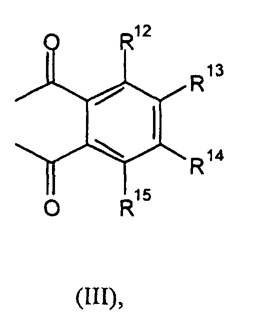 in which R¹², R¹³, R¹⁴ and R¹⁵ independently of one another, identically or differently, are in each case H, OR⁷, where R⁷ has the meaning mentioned, or CₙH₂ₙ₊₁, or CₙH₂ₙ₋₁, where n has the abovementioned meaning, and
R⁵, R⁶, R⁷ and R⁸ independently of one another, identically or differently, are in each case H, CₙH₂ₙ₊₁, or CₙH₂ₙ₋₁, where n has the abovementioned meaning, -C(O)R⁹ where R⁹ is equal to a linear or branched, optionally substituted alkyl or aryl radical, preferably a phenyl radical,
X, Y and Z independently of one another, identically or differently, are in each case =N-, =C(R¹⁶)- or -N(R¹⁷)- where R¹⁶ and R¹⁷ independently of one another, identically or differently, are in each case H or CₙH₂ₙ₊₁ or (CₙH₂ₙ)NR¹⁰R¹¹ having the abovementioned meanings,
or by the formula (II) 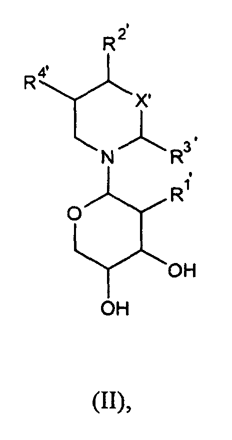 in which R^{1'} is equal to H, OH or Hal where Hal is equal to Br or Cl,
R^{2'}, R^{3'} and R^{4'} independently of one another, identically or differently, are in each case H, Hal where Hal is equal to Br or Cl, =O, CₙH₂ₙ₊₁ or OCₙH₂ₙ₋₁, or (CₙH₂ₙ)NR^{10'}R^{11'}, where R^{10'}, R^{11'}, independently of one another, have the abovementioned meaning of R¹⁰ and R¹¹, and
X' in each case is =N-, =C(R^{16'})- or -N(R^{17'})-, where R^{16'} and R^{17'} independently of one another have the abovementioned meaning of R¹⁶ and R¹⁷.

8. The process as claimed in one of claims 1-7, wherein the reaction is carried out at a temperature of about 40-70°C.

9. The process as claimed in one of claims 1-8, wherein the reaction is carried out in the presence of an acid.

10. The process as claimed in one of claim 9, wherein the acid is selected from p-toluenesulfonic acid, methanesulfonic acid, tetrafluoroboric acid, sulfuric acid, acidic ion exchangers and/or Lewis acids.

11. The process as claimed in one of claims 1-10, wherein the 3',4'-cyclic acetal of a pentopyranosyl nucleoside which is obtained is protected in the 2' position in a further step.

12. The process as claimed in claim 11, wherein the 2' position is protected by a protective group which is base-labile or can be removed by metal catalysis.

13. The process as claimed in claim 11 or 12, wherein the 2' position is protected by an acyl, acetyl, benzoyl, nitrobenzoyl and/or methoxybenzoyl group.

14. The process for the preparation of a pentopyranosylnucleoside as claimed in one of claims 11-13, wherein the 3',4'-cyclic acetal of a pentopyranosylnucleoside which is protected in the 2' position is deketalized.

15. The process as claimed in claim 14, wherein the deketalization is carried out in the presence of an acid, preferably in the presence of a strong acid.

16. The process as claimed in claim 14 or 15, wherein the reaction product obtained is worked up under dry basic conditions.

17. The process as claimed in claim 16, wherein the dry basic work-up is carried out in the presence of a solid hydrogencarbonate, carbonate and/or basic ion exchanger.

18. The process as claimed in one of claims 14-17, wherein the 4' position is protected in a further step.

19. The process as claimed in claim 18, wherein the 4' position is protected by an acid- or base-labile protective group.

20. The process as claimed in claim 19, wherein the 4' position is protected by a trityl group, DMT group, and/or by a β-eliminable group or an Fmoc group.

21. The process as claimed in one of claims 18-20, wherein a rearrangement of the protective group from the 2' position to the 3' position is carried out in a further step.

22. The process as claimed in claim 21, wherein the rearrangement is carried out in the presence of a base.

23. The process as claimed in claim 22, wherein the rearrangement is carried out in the presence of N-ethyldiisopropylamine and/or triethylamine.

24. A process for the preparation of a pentopyranosylnucleoside, which comprises
(a) reacting a protected nucleobase with a protected pentopyranose,
(b) removing the protective groups from the pentopyranosyl moiety of the product from step (a), and
(c) reacting the product from step (b) according to the process as claimed in one of claims 1-23.

25. The process as claimed in one of claims 21-24, wherein the pentopyranosylnucleoside obtained is phosphitylated or bonded to a solid phase in a further step.

26. A process for the preparation of a pentopyranosylnucleic acid, which comprises
(a) in a first step preparing a pentopyranosylnucleoside as claimed in one of claims 21-24,
(b) in a second step bonding the pentopyranosylnucleoside prepared according to step (a) to a solid phase, and
(c) in a further step extending the 3', 4'-protected pentopyranosylnucleoside bonded to a solid phase according to step b) by a phosphitylated 3', 4'-protected pentopyranosylnucleoside, and
(d) repeating step (c).

27. The process as claimed in claim 26, wherein at least one pentofuranosylnucleoside is also incorporated in step (b) and/or step (c).

28. The process as claimed in claim 26 or 27, wherein the coupling reagents employed for the extension according to step (c) are acidic activators.

29. The process as claimed in claim 28, wherein the acidic activators employed are p-nitrophenyltetrazole or benzimidazolium triflate.

30. The process as claimed in one of claims 26-29, wherein the protective groups and the oligomer formed are removed from the solid phase in a further step (e).

31. The process as claimed in claim 30, wherein the removal is carried out by hydrazinolysis.

32. The process as claimed in claim 30 or 31, wherein the oligomer obtained is purified by chromatography.

33. The process as claimed in claim 32, wherein the chromatographic purification is carried out on alkylsilylated silica gel.

34. The process as claimed in one of claims 28-33, wherein an allyloxy linker of the formula (IV)
S_{c1}NH(CₙH₂ₙ)CH(OPS_{c2}S_{c3})CₙH₂ₙS_{c4} (IV),
in which S_{c1} and S_{c4} independently of one another, identically or differently, in each case are a protective group selected from Fmoc and/or DMT,
S_{c2} and S_{c3} independently of one another, identically or differently, in each case are an allyloxy and/or diisopropylamino group and n is an integer from 1-12, is incorporated in a further step.

35. The process as claimed in claim 34, wherein the allyloxy linker 2-(S)-N-Fmoc-O¹-DMTO²-allyloxydiisopropylaminophosphinyl-6-amino-1,2-hexandiole is incorporated.

## Revendications

1. Procédé pour la préparation d'un acétal 3',4'-cyclique d'un pentopyranosyl-nucléoside, **caractérisé en ce qu'**on met à réagir un pentopyranosyl-nucléoside avec un aldéhyde, une cétone, un acétal ou cétal sous un vide partiel inférieur à 500 mbar.

2. Procédé selon la revendication 1, **caractérisé en ce qu'**on choisit l'aldéhyde parmi le formaldéhyde, l'acétaldéhyde, le benzaldéhyde ou le 4-méthoxybenzaldéhyde, l'acétal parmi le formaldéhyde-diméthylacétal, l'acétaldéhyde-diméthylacétal, le benzaldéhydediméthylacétal ou le 4-méthoxybenzaldéhyde-diméthylacétal, la cétone parmi l'acétone, la cyclopentanone ou cyclohexanone et le cétal parmi l'acétonediméthylcétal, le cyclopentanone-diméthylcétal, le cyclohexanonediméthylcétal ou sous la forme de 2-méthoxypropène.

3. Procédé selon la revendication 1 ou 2, **caractérisé en ce qu'**on purifie le pentopyranosyl-nucléoside avant la réaction.

4. Procédé selon la revendication 3, **caractérisé en ce qu'**on purifie le pentopyranosyl-nucléoside sur du SiO₂.

5. Procédé selon une des revendications 1-4, **caractérisé en ce qu'**on utilise comme pentopyranosyl-nucléoside un ribo-, arabino-, lyxo- ou xylo-pyranosyl-nucléoside.

6. Procédé selon une des revendications 1-5, **caractérisé en ce qu'**on utilise comme pentopyranosyl-nucléoside une pentopyranosyl-pyrine, -2,6-diaminopurine, -6-purinethiol, -pyridine, -pyrimidine, -adénosine, -guanosine, -isoguanosine, -6-thioguanosine, -xanthine, -hypoxanthine, -thymidine, -cytosine, -isocytosine, -indole, -tryptamine, -N-phtalotryptamine, -uracile, -caféine, -théobromine, -théophylline, -benzotriazole ou -acridine.

7. Procédé selon une des revendications 1-6, **caractérisé en ce qu'**on met à réagir un pentopyranosyl-nucléoside de formule (I) 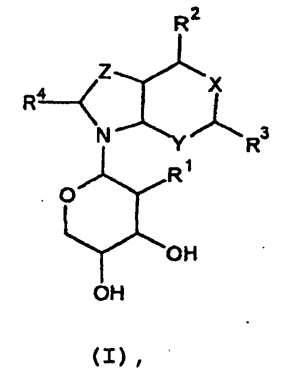 dans laquelle
R¹ représente H, OH ou Hal avec Hal égal à Br ou Cl,
R², R³ et R⁴ indépendamment les uns des autres, identiques ou différents, représentent respectivement H, Hal avec Hal égal à Br ou Cl, NR⁵R⁶, OR⁷, =O, CₙH₂ₙ₊₁ avec n étant un nombre entier de 1 à 12 ou (CₙH₂ₙ)NR¹⁰R¹¹ avec R¹⁰R¹¹ égal à H, CₙH₂ₙ₊₁ ou
R¹⁰R¹¹ liés sur un groupe de formule 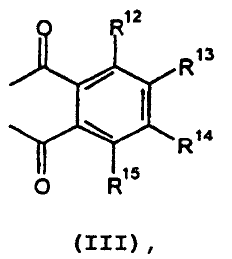 dans laquelle R¹², R¹³, R¹⁴ et R¹⁵ indépendamment les uns des autres, identiques ou différents, représentent respectivement H, OR⁷, dans laquelle R⁷ a la signification mentionnée, ou CₙH₂ₙ₊₁ ou CₙH₂ₙ₋₁, dans lesquelles n a la signification mentionnée ci-dessus et
R⁵, R⁶, R⁷ et R⁸ indépendamment les uns des autres, identiques ou différents, représentent respectivement H, CₙH₂ₙ₊₁ ou CₙH₂ₙ₋₁, dans lesquelles n a la signification mentionnée ci-dessus, C(O)R⁹ avec R⁹ étant un groupe alkyle, aryle, de préférence phényle, linéaire ou ramifié, éventuellement substitué,
X, Y et Z indépendamment les uns des autres, identiques ou différents, représentent respectivement =N-, =C(R¹⁶)- ou -N(R¹⁷)- avec R¹⁶ et R¹⁷ indépendamment les uns des autres, identiques ou différents, représentant respectivement H ou CₙH₂ₙ₊₁ ou (CₙH₂ₙ)NR¹⁰R¹¹ avec les significations mentionnées ci-dessus,
ou de formule (II) 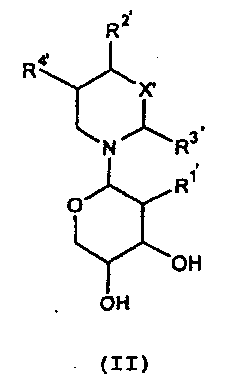 dans laquelle
R^{1'} représente H, OH ou Hal avec Hal égal à Br ou Cl,
R^{2'}, R^{3'} et R^{4'} indépendamment les uns des autres, identiques ou différents, représentent respectivement H, Hal avec Hal égal à Br ou Cl, =O, CₙH₂ₙ₊₁ ou OCₙH₂ₙ₋₁, ou (CₙH₂ₙ)NR^{10'}R^{11'}, dans laquelle R^{10'}, R^{11'} indépendamment l'un de l'autre ont la signification mentionnée ci-dessus pour R¹⁰ et R¹¹, et
X' représente respectivement =N-, =C(R^{16'})- ou -N(R^{17'})- dans lesquelles R^{16'} et R^{17'} indépendamment l'un de l'autre ont la signification mentionnée ci-dessus pour R¹⁶ et R¹⁷.

8. Procédé selon une des revendications 1 à 7, **caractérisé en ce qu'**on réalise la réaction à une température de 40 à 70 °C.

9. Procédé selon une des revendications 1 à 8, **caractérisé en ce qu'**on réalise la réaction en présence d'un acide.

10. Procédé selon la revendication 9, **caractérisé en ce que** l'acide est choisi parmi l'acide p-toluènesulfonique, l'acide méthanesulfonique, l'acide tétrafluoroborique, l'acide sulfurique, les échangeurs d'ions acides et/ou les acides de Lewis.

11. Procédé selon une des revendications 1 à 10, **caractérisé en ce que**, dans une autre étape on protège l'acétal 3',4'-cyclique d'un pentopyranosyl-nucléoside obtenu en position 2'.

12. Procédé selon la revendication 11, **caractérisé en ce qu'**on protège la position 2' par un groupe de protection clivable labile en milieu basique ou catalysé par un métal.

13. Procédé selon la revendication 11 ou 12, **caractérisé en ce qu'**on protège la position .2' par un groupe acyle, acétyle, benzoyle, nitrobenzoyle et/ou méthoxybenzoyle.

14. Procédé pour la préparation d'un pentopyranosyl-nucléoside selon une des revendications 11 à 13, **caractérisé en ce qu'**on décétalise l'acétal 3',4'-cyclique d'un pentopyranosyl-nucléoside protégé en position 2'.

15. Procédé selon la revendication 14, **caractérisé en ce qu'**on réalise la décétalisation en présence d'un acide, de préférence en présence d'un acide fort.

16. Procédé selon la revendication 14 ou 15, **caractérisé en ce qu'**on transforme le produit de réaction obtenu en milieu basique sec.

17. Procédé selon la revendication 16, **caractérisé en ce qu'**on réalise la transformation en milieu basique sec en présence d'hydrogénocarbonate solide et/ou d'échangeur d'ions basique.

18. Procédé selon une des revendications 14 à 17, **caractérisé en ce qu'**on protège la position 4' dans une autre étape.

19. Procédé selon la revendication 18, **caractérisé en ce qu'**on protège la position 4' par un groupe de protection labile en milieu acide ou basique.

20. Procédé selon la revendication 19, **caractérisé en ce qu'**on protège la position 4' par un groupe trityle, un groupe DMT, un groupe β-éliminable ou un groupe Fmoc.

21. Procédé selon une des revendications 18 à 20, **caractérisé en ce qu'**on réalise dans une autre étape un réarrangement du groupe de protection de la position 2' à la position 3'.

22. Procédé selon la revendication 21, **caractérisé en ce qu'**on réalise le réarrangement en présence d'une base.

23. Procédé selon la revendication 22, **caractérisé en ce qu'**on réalise le réarrangement en présence de N-éthyldiisopropylamine et/ou de triéthylamine.

24. Procédé pour la préparation d'un pentopyranosyl-nucléoside, **caractérisé en ce qu'**on
(a) met à réagir une nucléobase protégée avec un pentopyranose protégé,
(b) clive les groupes de protection de la partie pentopyranosyle du produit de l'étape (a), et
(c) met à réagir le produit de l'étape (b) conformément au procédé selon une des revendications 1 à 23.

25. Procédé selon une des revendications 21 à 24, **caractérisé en ce qu'**on réalise une phosphitation du pentopyranosyl-nucléoside obtenu dans une autre étape ou on le lie à une phase solide.

26. Procédé pour la préparation d'un acide pentopyranosyl-nucléique, **caractérisé en ce qu'**on
(a) prépare dans une première étape un pentopyranosyl-nucléoside selon une des revendications 21 à 24,
(b) lie dans une deuxième étape le pentopyranosyl-nucléoside préparé selon l'étape (a) à une phase solide, et
(c) rallonge dans une autre étape le pentopyranosyl-nucléoside 3'-,4'-protégé lié à une phase solide selon l'étape (b) pour obtenir un pentopyranosyl-nucléoside 3'-,4'-protégé transformé en phosphite, et
(d) répète l'étape (c).

27. Procédé selon la revendication 26, **caractérisé en ce qu'**on introduit à l'étape (b) et/ou l'étape (c) également au moins un pentofuranosyl-nucléoside.

28. Procédé selon la revendication 26 ou 27, **caractérisé en ce qu'**on utilise comme réactif de couplage pour l'allongement selon l'étape (c) des activateurs acides.

29. Procédé selon la revendication 28, **caractérisé en ce qu'**on utilise comme activateurs acides le p-nitrophényltétrazol ou le triflate de benzimidazolium.

30. Procédé selon une des revendications 26 à 29, **caractérisé en ce qu'**on sépare dans une autre étape (e) les groupes de protection et l'oligomère formé de la phase solide.

31. Procédé selon la revendication 30, **caractérisé en ce qu'**on réalise le clivage par hydrazinolyse.

32. Procédé selon la revendication 30 ou 31, **caractérisé en ce qu'**on purifie l'oligomère obtenu par chromatographie.

33. Procédé selon la revendication 32, **caractérisé en ce qu'**on réalise la purification chromatographique sur du gel de silice alkylsilylé.

34. Procédé selon une des revendications 28 à 33, **caractérisé en ce qu'**on introduit dans une autre étape un groupe liant allyloxy de formule (III)
S_{c1}NH(CₙH₂ₙ)CH(OPS_{c2}S_{c3})CₙH₂ₙS_{c4} (III),
dans laquelle S_{c1} et S_{c4} indépendamment l'un de l'autre, identiques ou différents, représentent respectivement un groupe de protection choisi parmi Fmoc et/ou DMT,
S_{c2} et S_{c3} indépendamment l'un de l'autre, identiques ou différents, représentent respectivement un groupe allyloxy et/ou diisopropylamino et n un nombre entier de 1 à 12, de préférence de 1 à 8, en particulier de 1 à 4.

35. Procédé selon la revendication 34, **caractérisé en ce qu'**on introduit comme groupe de liaison allyloxy le 2-(S)-N-Fmoc-O¹-DMT-O²-allyloxydiisopropylaminophosphinyl-6-amino-1,2-hexanediol.
